# EUROPÄISCHE PATENTANMELDUNG

(11) **EP 2 698 095 A1**
(43) Veröffentlichungstag der Anmeldung: **19.02.2014**
(21) Anmeldenummer: 12005893.8
(22) Anmeldetag: 16.08.2012
(51) Int. Cl.: A61B 1/01, A61B 1/31, A61B 1/00

(54) **Vorrichtungen zum Halten eines Patienten während einer endoskopischen Untersuchung**

(71) Anmelder: Neumann, Martin, Dr., 91080 Uttenreuth (DE)
(72) Erfinder: Neumann, Martin, Dr., 91080 Uttenreuth (DE)
(74) Vertreter: Gosdin, Michael

(57) **Zusammenfassung**

Die Erfindung betrifft eine Vorrichtung (1) zum Halten eines Patienten während einer endoskopischen Untersuchung, umfassend: ein Trägerelement (2), ein am Trägerelement (2) angeordnetes Halteelement (3), wobei ein proximales Ende (4) des Halteelements (3) am Trägerelement (2) befestigt ist und wobei an einem distalen Ende (5) des Halteelements (3) eine Rückenstütze (6) befestigt ist, mindestens einen Befestigungsgurt (7) der mit seinen Enden (8, 9) an der Rückenstütze (6) lösbar befestigbar ist und den Bauch eines Patienten umfasst und mindestens eine Druckplatte (10), die mittels des mindestens einen Befestigungsgurts (7) umfasst und gegen die Rückenstütze (6) verspannt werden kann. Um in verbesserter Weise das Endoskopieren, insbesondere als Koloskopie, durchfahren zu können, sieht die Erfindung weiter vor, dass auf der dem Bauch des Patienten zugewandten Seite der Druckplatte (10) mindestens ein in seinem Volumen veränderbares Polsterelement (13) angeordnet ist.

## Beschreibung

Die Erfindung betrifft eine Vorrichtung zum Halten eines Patienten während einer endoskopischen Untersuchung.

Für diagnostische und therapeutische Eingriffe wird die Endoskopie eingesetzt, bei der flexible Endoskope verwendet werden. Diese Vorgehensweise ist im Stand der Technik hinlänglich bekannt. Das Endoskop wird rektal oder oral eingeführt und an die zu untersuchende Stelle bzw. entlang des zu untersuchenden Bereichs geschoben, z. B. in den Bereich des Dickdarms (Koloskopie), des Zwölffingerdarms, des Magens (Gastroskopie) oder der Speiseröhre.

Die Richtungssteuerung der in zwei Ebenen bewegbaren Endoskopspitze erfolgt dabei mechanisch mittels zweier Steuerräder am Endoskophandgriff, um möglichst lumenzentriert und die Darmwand schonend vorzuspiegeln oder um eine Zielregion optimal einzustellen. Der Vorschub bzw. die Rückwärtsbewegung des Endoskops kann dabei manuell durchgeführt werden.

Beim Endoskopieren ist es sehr vorteilhaft, wenn der Patient während der Untersuchung in der Seitenlage verharrt. Dies ist vorteilhaft, weil insbesondere die Koloskopie wegen der Zugänglichkeit des Rektums stets in der (Links-)Seitenlage beginnt. Wird - was bevorzugt ist - für den Vortrieb des Endoskops ein motorischer Antrieb eingesetzt, muss die Seitenlage des Patienten beibehalten werden.

Dies hat gewisse Probleme zur Folge. Im Falle der Koloskopie liegen die Darmschlingen quasi in unveränderlicher Position. Während bei der klassischen Koloskopie im Falle dessen, dass eine Darmschlinge ungünstig für den weiteren Vortrieb des Endoskops liegt, der Patient temporär auf den Rücken gelegt werden kann, um aufgrund der Schwerkraft die Darmschlinge eine andere Position einnehmen zu lassen, ist dies dann nur schwerlich möglich, wenn für die Endoskopie ein motorischer Endoskopantrieb eingesetzt wird. Der Patient muss dann in der Seitenlage verbleiben.

Ferner muss beim Einsatz des genannten Endoskopantriebs sichergestellt sein, dass der Patient relativ zum Antriebssystem für das Endoskop unbeweglich ist. Bei Relativbewegungen zwischen dem Endoskopantrieb und dem Patienten kommt es unweigerlich zu störenden Abweichungen der Ist-Lage des Endoskops zur seiner Soll-Lage.

Die bevorzugt eingesetzten motorischen Endoskopantriebe sind beispielsweise in der DE 10 2005 002 461 B3 beschrieben. Mit dieser Antriebsvorrichtung ist es möglich, auch bei schwierigen Untersuchungsverhältnissen die Vorschubbewegung des Endoskops feinfühlig zu bewerkstelligen und die Kontrolle über die für den Vorschub erforderlichen Kräfte zu haben, um unnötige Schmerzen, Schäden an der Darmwand oder gar Darm-Perforationen zu vermeiden.

Um bei der Koloskopie das Endoskop bis zum Zökalpol (Übergang vom Dünndarm in den Dickdarm) vorwärts zu bewegen bzw. für die genaue Diagnostik wieder zurück zu bewegen, muss bisher das Endoskop von einer Schwester (bzw. vom Assistenzpersonal) oder vom Untersucher selbst vorgeschoben bzw. geführt werden. Beide Möglichkeiten haben allerdings Nachteile. Im Falle des Einsatzes der Schwester für das Vorschieben des Endoskops ist für diese Aufgabe Personal gebunden; das Zusammenspiel zwischen Arzt und Assistenzpersonal ist im übrigen für die spezielle Aufgabe nicht immer ideal gegeben. Wenn der Untersucher selber das Endoskop schiebt, braucht er dazu eine Hand, die ihm dann aber fehlt, um ein zweites Steuerrad des Endoskops oder um einen Arbeitskanal des Endoskops zu bedienen.

Mit dem genannten motorischen Endoskopantrieb steht ein System zur Verfügung, mit dem bei der Endoskopie, speziell bei der Koloskopie, das Endoskop anstatt mit der Hand elektronisch über einen Fußschalter vor und zurück bewegt werden kann. Eine druckkontrollierte Abschaltautomatik verhindert, dass ein zu großer Druck auf die Darmwand einwirkt. Damit können bei der Koloskopie mehr diagnostische Sicherheit, Präzision, Effizienz und Komfort erreicht werden. Vor allem hat der Untersucher eine Hand für die Richtungssteuerung der Endoskopspitze und für die Betätigung der Instrumente über den Arbeitskanal frei.

Ein generelles Problem ist hierbei allerdings, dass sich das Endoskop beim Einschieben aufschieben kann, d. h. durch den Vorschub kann sich ein Darmabschnitt (zumeist im Bereich des Sigmas) als Schlinge aufschieben und verhindern, dass sich trotz Vorschub die Endoskopspitze (Optik) nicht voran bewegt. In diesem Fall muss durch zusätzliche Manöver (insbesondere durch Begradigungsmanöver bzw. eine äußere Schienung) versucht werden, für das Endoskop wieder die notwendige Abstützung zu erreichen, damit der Vorschub auch bei der Endoskopspitze ankommt.

Nach der konventionellen Methode hilft dabei Zurückziehen des Endoskops zwecks Begradigen und ein erneutes Vorschieben, oftmals wird dabei der Endoskopschaft gedreht, um mit einer Rotation eine andere bzw. bessere Positionierung bzw. Abstützung zu erreichen.

Häufig wird dabei zusätzlich von außen manuell geschient, d. h. die Endoskopie-Assistenz drückt mit der Hand bzw. mit den Fingern in den Bauch des Patienten, um ein Widerlager von außen zu schaffen, vor allem im Bereich der ersten "Darmkurve" (Projektion auf den Nabelbereich). Meistens erfolgt dies noch an einer zweiten Stelle im linken unteren Bauchbereich, um auch hier ein Widerlager von außen für das eingeführte Endoskop zu geben.

Wenn das alles nicht hilft, kann der Patient noch auf den Rücken gedreht werden, damit das Gewicht des Bauchs auf den Darm drückt, der dabei günstiger zu liegen kommen kann.

Insgesamt sind dies allerdings nachteilig vor allem für das Assistenzpersonal anstrengende und zeitraubende Manöver, um das Zökum (Übergang vom Dickdarm zum Dünndarm) als Ziel beim Hochspiegeln zu erreichen. Im übrigen ist dies auch für den Patienten unangenehm, sofern dieser nicht komplett sediert ist. Ist er tief sediert, fehlt es wiederum an der Möglichkeit der Mitarbeit durch den Patienten, d. h. der Patient kann bei den Umlager-Aktionen überhaupt nicht mithelfen, was es wiederum für das Assistenzpersonal schwer macht.

Der Erfindung liegt im Lichte der vorstehenden Problematik die **Aufgabe** zugrunde, eine Vorrichtung zum Halten eines Patienten während einer endoskopischen Untersuchung vorzuschlagen, mit der in verbesserter Weise das Endoskopieren, insbesondere als Koloskopie, durchgeführt werden kann. Hierbei soll es insbesondere möglich sein, das Endoskopieren unter Einsatz des genannten Endoskopantriebs sicher und ohne hohe Belastung für den Patienten vornehmen zu können. Dabei soll speziell sichergestellt werden, dass es zu keinen unerwünschten Relativbewegungen zwischen Endoskopantrieb und Patient kommt. Der Patient soll dabei stabil in der Seitenlage gehalten werden, wobei es möglich sein soll, einen einstellbaren, variablen Widerstand auf den Bauch des Patienten auszuüben, um das Hochspiegeln des Endoskops zu erleichtern. Ein wichtiger Aspekt ist dabei, dass der Bauch des Patienten gezielt abgestützt werden kann, was in angenehmer und komfortabler Weise für den Patienten erfolgen soll.

Die **Lösung** dieser Aufgabe durch die Erfindung ist dadurch gekennzeichnet, dass die Vorrichtung folgende Komponenten umfasst:
- ein Trägerelement,
- ein am Trägerelement angeordnetes Halteelement, wobei ein proximales Ende des Halteelements am Trägerelement befestigt ist und wobei an einem distalen Ende des Halteelements eine Rückenstütze befestigt ist,
- mindestens einen Befestigungsgurt der mit seinen Enden an der Rückenstütze lösbar befestigbar ist und den Bauch eines Patienten umfasst und
- mindestens eine Druckplatte, die mittels des mindestens einen Befestigungsgurts umfasst und gegen die Rückenstütze verspannt werden kann, wobei auf der dem Bauch des Patienten zugewandten Seite der Druckplatte mindestens ein in seinem Volumen veränderbares Polsterelement angeordnet ist.

Das Polsterelement ist bevorzugt als aufblasbare Luftmanschette ausgebildet. Es kann mit einer Druckquelle für ein Fluid (Flüssigkeit oder Gas), insbesondere für Luft, in Verbindung stehen. Die Druckquelle ist dabei bevorzugt eine handbetätigte Luftpumpe. Alternativ kann auch vorteilhaft eine elektrisch betätigte Luftpumpe eingesetzt werden.

An der Druckplatte kann mindestens eine Klemmspange angeordnet sein, mit der die seitliche Erstreckung des Polsterelements begrenzt werden kann. Die mindestens eine Klemmspange kann dabei in der Ebene der Druckplatte verschieblich angeordnet sein.

Weiterhin sieht eine Fortbildung vor, dass mindestens zwei Polsterelemente nebeneinander auf der Druckplatte angeordnet sind. Für jedes Polsterelement kann eine Klemmspange vorhanden sein, mit der die seitliche Erstreckung des Polsterelements begrenzt werden kann. Zwischen zwei Polsterelementen kann ein Abstandselement angeordnet sein, das die beiden Polsterelemente voneinander beabstandet hält.

Auf der dem Bauch des Patienten zugewandten Seite des Polsterelements kann ein Druckübertragungselement angeordnet sein. Das Druckübertragungselement kann eine runde oder elliptische Form aufweisen, gesehen senkrecht auf die Druckplatte. Es kann zumindest in seiner Kontaktfläche zum Bauch des Patienten aus elastischem oder gelartigem Material bestehen. Das Druckübertragungselement kann auf dem Polsterelement aufgeklebt sein.

An der Druckplatte oder an der Rückenstütze kann eine Trägerplatte angeordnet sein, die zur Halterung der mindestens einen Druckquelle, insbesondere der mindestens einen Luftpumpe, ausgebildet ist. An der Trägerplatte kann mindestens eine Halteklemme für die Druckquelle, insbesondere für die Luftpumpe, angeordnet sein.

Die Druckplatte hat bevorzugt eine ovale Form. Sie kann an der dem Bauch des Patienten zugewandten Seite eine konkave Form aufweisen. Die Druckplatte besteht bevorzugt aus Plexiglas.

Bevorzugt hat die Druckplatte mindestens zwei, sich aus der Grundkontur der Druckplatte erhebende Haltenoppen, mit denen das Abrutschen des Befestigungsguts verhindert wird. Die Druckplatte kann auch vier oder sechs Haltenoppen aufweisen, die gespiegelt zur Längsachse der Druckplatte im Randbereich der Druckplatte angeordnet sind.

Eine Weiterbildung sieht zwei parallel zueinander verlaufende Befestigungsgurte vor. Der mindestens eine Befestigungsgurt kann als im Querschnitt flaches Textilgut ausgebildet sein. Er kann einen öffenbaren Verschluss aufweisen. Er kann mit seinem Ende an mindestens einer Haltestange eingehängt sein, die an der vom Patienten abgewandten Seite der Rückenstütze angeordnet ist. Bevorzugt sind zwei parallel zueinander verlaufende Haltestangen an der Rückenstütze angeordnet.

Die Vorrichtung hat vorzugsweise weiterhin einen Befestigungsgurt, mit dem das Trägerelement und die Rückenstütze miteinander verspannt werden können.

Das Trägerelement kann auf Rollen angeordnet sein, wobei die Rollen feststellbar sind. Das Halteelement kann in seinen Enden als gekröpfte Stange ausgeführt sein, deren Endabschnitt um eine vertikale Achse relativ zur Trägerelement bzw. zur Rückstütze drehbar angeordnet ist. Der Endabschnitt des Halteelements kann relativ zum Trägerelement drehfestgelegt werden, insbesondere mit einem mit einer Schraube versehenen Handrad. Der Endabschnitt des Halteelements kann auch relativ zur Rückenstütze drehfestgelegt werden, insbesondere mit einem mit einer Schraube versehenen Handrad.

Am Trägerelement kann weiterhin ein Tragarm angeordnet sein, der zum Halten einer Endoskopvorschubvorrichtung ausgebildet ist.

Das Eingeben von Druckluft in das mindestens eine Polsterelement sowie das Ablassen von Druckluft aus demselben kann - wie oben erwähnt - durch eine handbetätigte Luftpumpe erfolgen, wie sie im medizinischen Bereich für das Aufpumpen von Blutdruckmanschetten bekannt sind.

Eine weiterentwickelte Lösung sieht allerdings diesbezüglich eine maschinelle Unterstützung vor, die dem Untersuchenden gegebenenfalls sehr hilfreich unterstützt. Hiernach ist die Luftpumpe elektrisch betätigt und kann von einer zentralen Bedienstation bzw. von der Rückenplatte aus, betätigt werden. Der Untersuchende kann durch Betätigung eines entsprechenden Schalters oder Knopfes (Aufblas-Taster "Insuflate") Luft in das mindestens eine Polsterelement eingeben, bis ein gewünschter Inflationsgrad erreicht ist. Bei Bedarf kann so auch in einfacher Weise dosiert nachgepumpt werden.

Genauso kann das Ablassen von Luft aus dem mindestens einen Polsterelement erfolgen. Hiernach sind vorzugsweise Magnetventile in der pneumatischen Leitung zum Polsterelement angeordnet, über die - wiederum betätigt durch einen Schalter oder einen Knopf (Ablass-Taster "Desuflate") von der zentralen Bedienstation oder von der Rückenplatte aus - Luft aus dem Polsterelement abgelassen werden kann. Besagte Steuerelemente für das Aufblasen und Ablassen von Luft können also auch an der Rückstütze angeordnet sein.

Das System zum Aufblasen und Ablassen von Luft der Polsterelemente sieht gemäß einer besonders bevorzugten Ausführungsform der Erfindung für zwei Polsterelemente zwei elektrische Luftpumpen, zwei Drucksensoren und zwei Magnetventile vor, die Elemente für die Bedienung können in einem Bedienfeld an der Rückenstütze angeordnet sein. Die Bedienung kann über zwei Taster "Insuflate" ("Luft ein") und zwei Taster "Desuflate" ("Luft aus") erfolgen. Weiterhin können zwei Druckanzeigedisplays für die beiden Polsterelemente vorgesehen werden.

Die elektrische Pumpeneinheit kann in einem Gerätekasten am Rollstativ untergebracht werden.

Der im mindestens einen Polsterelement herrschende Druck kann also per Drucksensor gemessen und an einem Display der zentralen Bedienstation angezeigt werden.

Während das Trägerelement - wie erläutert - zumeist ein mobiles Stativ ist, sieht eine alternative Lösung vor, dass das Trägerelement als ein an einer Untersuchungsliege befestigter bzw. befestigbarer Kloben ausgebildet ist. Dieser Kloben kann an einem Rahmenteil der Untersuchungsliege angeschraubt oder in anderer Weise festgelegt werden und die Halterung für das Halteelement aufweisen, wobei letzteres dann wiederum mit der Rückenstütze verbunden ist. Der Kloben lässt dabei bevorzugt auch eine Höheneinstellung des Trägerelements zu.

Mit der vorgeschlagenen Ausstattung wird es möglich, insbesondere das Hochspiegeln des Endoskops unter Einsatz eines motorischen Endoskopantriebs zu verbessern. Vorteilhaft sind die einfache Anwendbarkeit der Vorrichtung und ihre hohe Wirksamkeit.

Die Druckplatte der Vorrichtung simuliert dabei im gegebenen Falle die Wirkung der Schwerkraft, so als würde der Patient in die Rückenlage gedreht, wodurch das Hochspiegeln des Endoskops erleichtert wird, obwohl der Patient in der Seitenlage verbleibt.

Die Druckplatte wirkt wie eine äußere Schienung der Bauchwand. Die Wölbung der Druckplatte hat die Wirkung wie eine von einer Krankenschwester aufgelegte Hand. Dies ist für den Patienten beim Endoskopieren angenehm. Die Größe der Druckplatte wird aus einer verfügbaren Auswahl entsprechend ausgewählt.

Demgemäß bietet der erfindungsgemäße Aufbau eine große Hilfe, da zum einen der Patient stabil gelagert und gesichert werden kann und gleichzeitig der Bauch mit der vorgeschlagenen Bauch-Druckplatte im Sinne einer äußeren Schienung angespannt werden kann. Dadurch wird ein gleichmäßiger Druck von außen auf das Abdomen erzeugt (äußere Schienung).

Mit dieser Ausgestaltung des Systems kann bereits zu Beginn der Untersuchung ein flächiger Druck auf den Bauch aufgebaut werden, so dass deutlich verbesserte Resultate bei der Hochspiegelrate erzielt werden. Die Druckplatte (Bauchplatte) wird dabei mit zwei Gurten, die über die Platte laufen, angezogen. Gegenlager bildet die Rückenstütze, an der die Spanngurte befestigt sind.

Der Andruck auf den Bauch des Patienten kann dabei flächig erhöht bzw. abgesenkt werden, was durch einfaches Aufpumpen bzw. Ablassen von Luft aus dem Polsterelement (Balgmanschette) erfolgen kann. Dies ist bei einem einfachen Handling möglich. Insbesondere ist dies einfacher zu bewerkstelligen, als die Spanngurte nachzuziehen. Der Druck kann gezielt an zwei oder mehr Bereichen der Druckplatte aufgebaut werden, insbesondere im medialen Bereich (Bauchnabelposition) und im lateralen Bereich der Platte, was durch das genannte bevorzugte Zwei-Kammersystem mit zwei Zuleitungen (Schläuchen) möglich wird.

Durch den permanenten Andruck der Druckplatte, die mit den Befestigungsgurten mit der Rückenstütze verspannt ist, ergibt sich von Anfang der Endoskopie an eine geringere Darm-Schlingenbildung und somit ein geringeres Aufschieben des Endoskops.

Der Patient ist in einer gesicherten, stabilen Lage, ein Herunterfallen von der Untersuchungsliege ist ausgeschlossen.

Es sei bemerkt, dass sich das vorgeschlagene Konzept auch in dem Falle bewährt hat und vorgeschlagen wird, wenn ein händischer Vorschub des Endoskops erfolgt, also der motorische Endoskopantrieb nicht eingesetzt wird.

Das Trägerelement ist bevorzugt als Rollstativ ausgebildet. Es können Gewichte in den Auslegern als Sicherung gegen ein Umkippen vorgesehen werden. Der Befestigungsgurt zwischen Trägerelement und Rückenstütze verhindert ein Wegrutschen des Trägerelements (Stativs) von der Untersuchungsliege und bannt damit die Gefahr des Herunterfallens des Patienten von der Untersuchungsliege. Die Rückenstütze ist höhenverstellbar an der genannten Winkelstange angeordnet. Durch Verdrehen der Winkelstange lässt sich der Abstand zum Patientenrücken angleichen. Durch eine Rotationsmöglichkeit in der Halterung lässt sich die Rückenstütze an den Patientenrücken-Winkel anpassen.

Die Haltestangen an der Rückseite der Rückenstütze können herausnehmbar sein, um in einfacher Weise die Gurtschlaufen der Befestigungsgurte aufnehmen zu können. Die Befestigungsgurte lassen sich leicht in Achsrichtung der Haltestangen verschieben und so optimal positionieren.

Die Befestigungsgurte sind mit Schnellverschlüssen lösbar, was von Vorteil ist, wenn der Patient auf den Rücken gedreht werden muss. Die Gurte sind auch an ihrer unteren Aufnahme lösbar, damit können die Gurte komplett entfernt und gewaschen werden.

Die Druckplatten sind bevorzugt transparent, um gegebenenfalls die Lichtdurchscheinung (Diaphanoskopie) des Endoskops sehen zu können. Sie können mehrere Randerhöhungen aufweisen, um das Abrutschen der Gurte zu verhindern.

Der Patient kann somit in definierter Position gesichert werden. Ein Wegrutschen von der eingestellten Position des Endoskopantriebs ist ausgeschlossen.

Es sind weiterhin eine Erleichterung und Verbesserung der Hochspiegel-Rate des Endoskops durch einen primär angelegten Druck von außen auf die Bauchwand gegeben. Der Spanngurt kann jederzeit gelöst und abgelegt werden, damit in schwierigen Fällen die Schwester zu jedem Zeitpunkt mit geübten Fingerspitzen schienen kann. Durch den flächigen Andruck der Druckplate (von z. B. 100 N) ergibt sich von vornherein ein vergleichbarer Effekt wie durch die Schwerkraft.

Von Vorteil ist weiterhin, dass keine Aktion durch das Umlagern des Patienten auf den Rücken erforderlich ist, was eine Entlastung für das Assistenzpersonal bedeutet und weniger Personal bindet.

Der Arzt kann selber eine Grundspannung durch entsprechende Aufgabe von Druckluft auf das Polsterelement anlegen; auch ein Nachspannen, Entspannen und Lösen der Spanngurte ist zusätzlich möglich. Generell empfiehlt sich ein Entspannen bzw. Lösen nach dem Erreichen des Zökalpols, so dass dann ein entspanntes Zurückspiegeln möglich ist.

Ein tableau-ähnliches Element (s. die oben genannten Trägerplatte) an der patientenabgewandten Seite der Rückenstütze mit Beschriftung (Bauchskizze und Markierung des medialen Ballons und des lateralen Ballons) dient als Halteelement für die beiden Handbälge und Manometer bzw. als Arretierungselemente für die Parkposition der beiden Zuleitungsenden. Wenn sie nicht an die Platte angeschlossen sind, können sie dort aufgesteckt werden. Des weiteren können hier auch gegebenenfalls Schalter und Kontrollanzeigen für eine motorangetriebene Luftpumpe vorgesehen werden, wie es beispielsweise für elektrische Blutdruckmessgeräte bekannt ist.

Vorgesehen werden können ferner zwei separate oder auch ein geteiltes aufblasbares Manschettenelement, das lösbar an der Unterseite der gewölbten Bauchplatte fixierbar ist, beispielsweise mit Ecklaschen an den Kammern, die über Erhebungen an der Plattenoberseite gelegt werden können.

Die genannte Klemmspange kann vorgesehen werden, um eine große Luftkammer in zwei Kammern zu teilen. Es können auch zwei Spangen vorgesehen werden, die von der Mitte aus nach außen geschoben werden können, um einen vorwölbaren Luftkammerabschnitt an die Ränder zu drücken.

Statt der genannten Klemmspangen kann auch - wie ebenfalls bereits erwähnt - ein Abstandselement in Form eines Mittenelement vorgesehen werden, das von unten an der Druckplatte den Mittenbereich der Luftkammer zusammendrückt. Je nach Breite des Mittenbereichs bleibt der Randbereich frei, in dem sich die Luftkammer vorwölben kann.

Vorgesehen werden können ferner zwei Handblasebälge (Luftpumpen) mit Manometer und zwei Zuleitungen, die mit der Manschette verbindbar sind (Konnektoren, gegebenenfalls Ventile, die durch die Druckplatte geführt sind), so dass ein einfacher Anschluss ermöglich wird und auch in einfacher Weise wieder diskonnektiert werden kann.

Gegebenenfalls kann - um den lokalen Druck auf den Bauch zu verstärken - auch vorgesehen werden, ein härteres Druckübertragungselement ("Sohle") mit Vorwölbung auf das Polsterelement (Manschette) zu kleben.

Beim Endoskopieren kann der Fall auftreten, dass durch das Aufschieben des Endoskops ein stärkerer Druck von Inneren des Kolons auf die Bauchoberfläche ausgeübt wird. Klassisch wird dem so begegnet, dass eine Hilfskraft händisch von außen auf die Bauchdecke einen Gegendruck aufgibt. Die vorliegende Erfindung bietet diesbezüglich eine besonders vorteilhafte Möglichkeit, besagte Operation der Hilfskraft zu ersetzen.

Hierzu ist zwischen dem Polsterelement und der Bauchoberfläche ein Drucksensor angeordnet; im Falle einer Anzahl an Polsterelementen ist eine entsprechende Anzahl an Drucksensoren vorgesehen. Der Drucksensor erfasst einen ansteigenden Druck, der vom Inneren des Körpers heraus auf die Bauchdecke ausgeübt wird. Die genannten Drucksensoren stehen mit einer Steuerung in Verbindung. Diese kann nun automatisch veranlassen, dass der Luftdruck in dem entsprechenden Polsterelement, dem der Drucksensor zugeordnet ist, erhöht wird, um temporär und lokal einen erhöhten Gegendruck von außen auf die Bauchdecke auszuüben.

Werden mehrere pneumatisch aufblasbare Polsterelemente mit jeweiligen Sensoren eingesetzt, kann entsprechend feinmaschig ein lokaler Druckanstieg erfasst und genau so gezielt und lokal begrenzt ein Gegendruck aufgebaut werden. Hierfür werden bevorzugt mindestens zwei, besonders bevorzugt mehr als zwei Polsterelemente vorgesehen.

In der Zeichnung sind Ausführungsbeispiele der Erfindung dargestellt. Es zeigen:
- Fig. 1: in perspektivischer Darstellung schematisch einen Patienten auf einer Untersuchungsliege, wobei im Rückenbereich des Patienten eine Vorrichtung zum Halten des Patienten während einer anstehenden endoskopischen Untersuchung positioniert ist,
- Fig. 2: in perspektivischer Darstellung einen angedeuteten Bereich des Bauchs des Patienten mit einer Druckplatte und Befestigungsgurten, die Bestandteil der Vorrichtung zum Halten des Patienten sind,
- Fig. 3: die Ansicht aus der Richtung "A" gemäß Fig. 2, wobei ein Teil des Patienten und die Druckplatte der Vorrichtung zu sehen ist,
- Fig. 4: eine zu Fig. 3 alternative Ausgestaltung der erfindungsgemäßen Vorrichtung und
- Fig. 5: die perspektive Darstellung der Rückseite einer Rückenstütze der Vorrichtung.

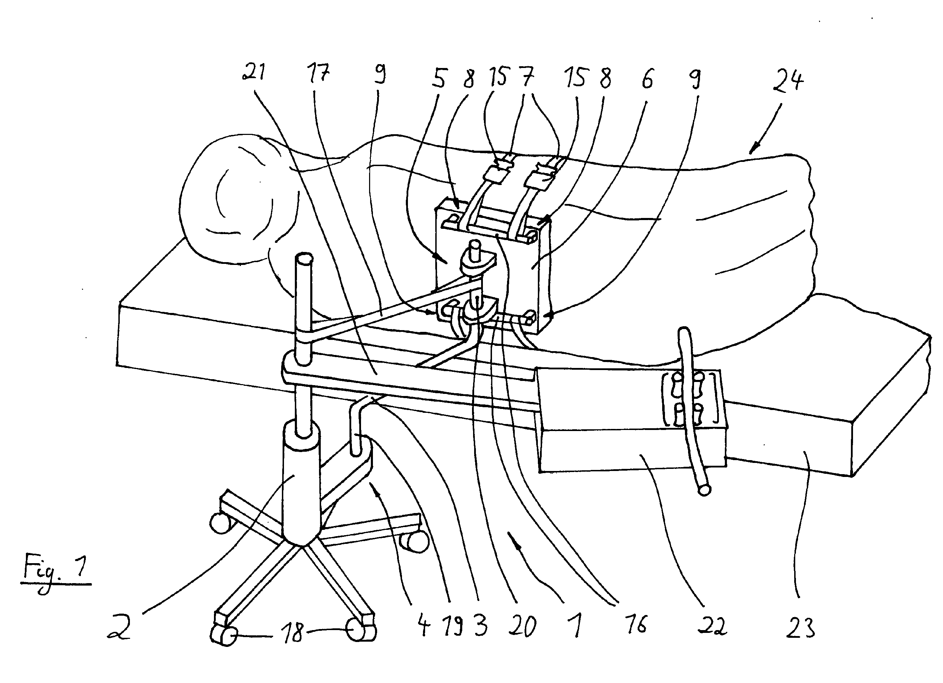

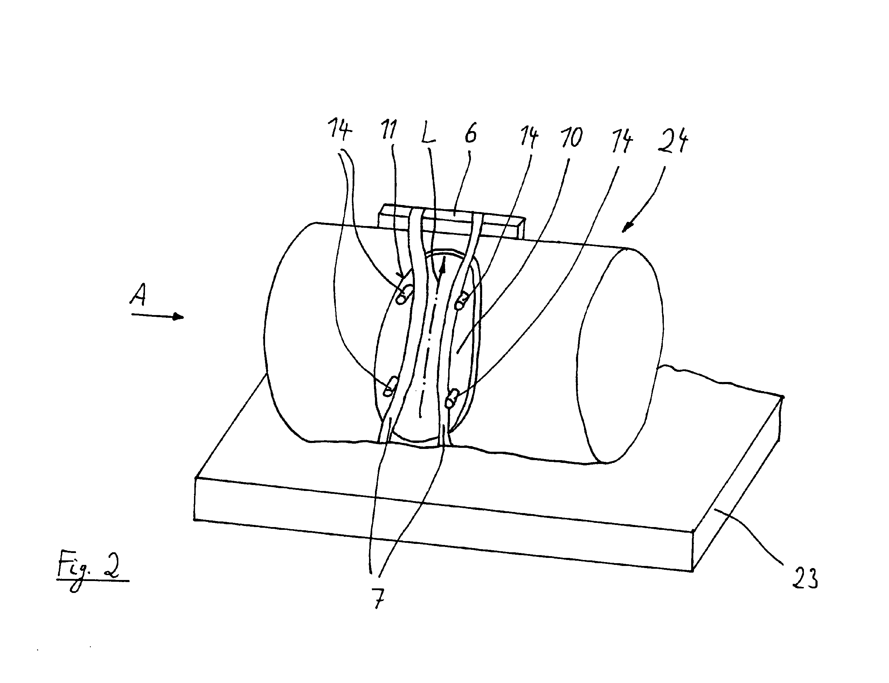

In den Figuren 1 und 2 ist ein Patient 24 auf einer Untersuchungsliege 23 in verschiedenen Ansichten dargestellt. An der Untersuchungsliege 23 ist im Rücken des Patienten 24 eine Vorrichtung 1 zum Halten des Patienten angeordnet. Die Vorrichtung 1 umfasst zunächst ein Trägerelement 2, das auf feststellbaren Rollen 18 angeordnet ist. Am Trägerelement 2 ist ein Halteelement 3 mit seinem proximalen Ende 4 befestigt. Bei dem Halteelement 3 handelt es sich um eine in den Endabschnitten 19 und 20 gekröpfte Stange, die um die vertikale Achse drehbar angeordnet ist. Das distale Ende 5 des Halteelements 3 ist an einer plattenartigen Rückenstütze 6 befestigt. Durch Wahl der Position des Halteelements (und anschließendes Fixieren des Halteelements relativ zum Trägerelement 2 bzw. zur Rückenstütze 6 mit nicht dargestellten Befestigungsschrauben) kann der effektive Abstand zwischen Trägerelement 2 und Rückenstütze 6 eingestellt werden.

Weiterhin sind zwei Befestigungsgurte 7 vorgesehen, die mit ihren Enden 8 bzw. 9 an der vom Patienten 24 abgewandten Rückseite der Rückenstütze 6 befestigt sind. Hierzu sind an der Rückseite der Rückenstütze 6 zwei parallele Haltestangen 16 angeordnet, in die die schlaufenartigen Enden der Befestigungsgurte 7 eingehängt werden können.

Die Befestigungsgurte 7 können mit einer schlauchartigen, auswechselbaren Kunststoffhülle eingefasst werden, um hohen hygienischen Ansprüchen gerecht zu werden.

Wie in Fig. 1 weiter zu sehen ist, ist am Trägerelement 2 auch ein Tragarm 21 angeordnet, der an seinem Ende eine Endoskopvorschubvorrichtung 22 tragen kann. Durch eine Fixierung des Tragarms 21 relativ zum Trägerelement 2 (mit einer nicht dargestellten Schraubbefestigung) kann die Endoskopvorschubvorrichtung 22 relativ zum Patienten 24 in ihrer Lage festgelegt werden.

Damit die Rückenstütze 6 sich nicht unbeabsichtigt vom Trägerelement 2 entfernen kann, ist ein Befestigungsgurt 17 vorgesehen.

Die Befestigung des Patienten 24 an der Vorrichtung 1 geht aus Fig. 2 hervor. Während der Rücken des Patienten 24 an der Rückenstütze 6 anliegt, umfassen die Befestigungsgurte 7 den Bauch des Patienten 24. Im Bauchbereich ist dabei eine Druckplatte 10 auf dem Bauch des Patienten 24 aufgelegt und mit den Befestigungsgurten 7 mit der Rückenstütze 6 verspannt.

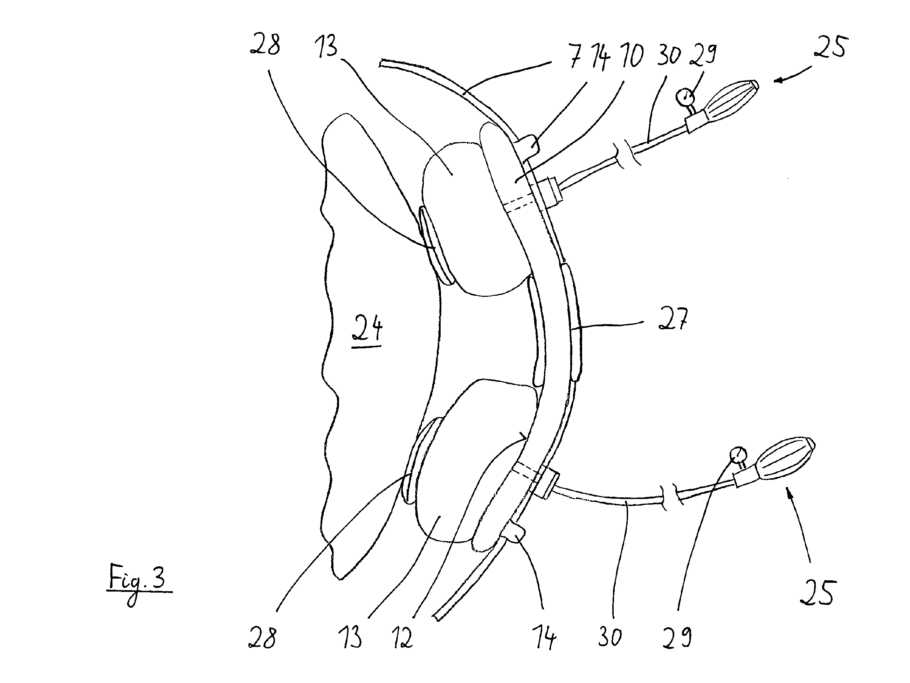

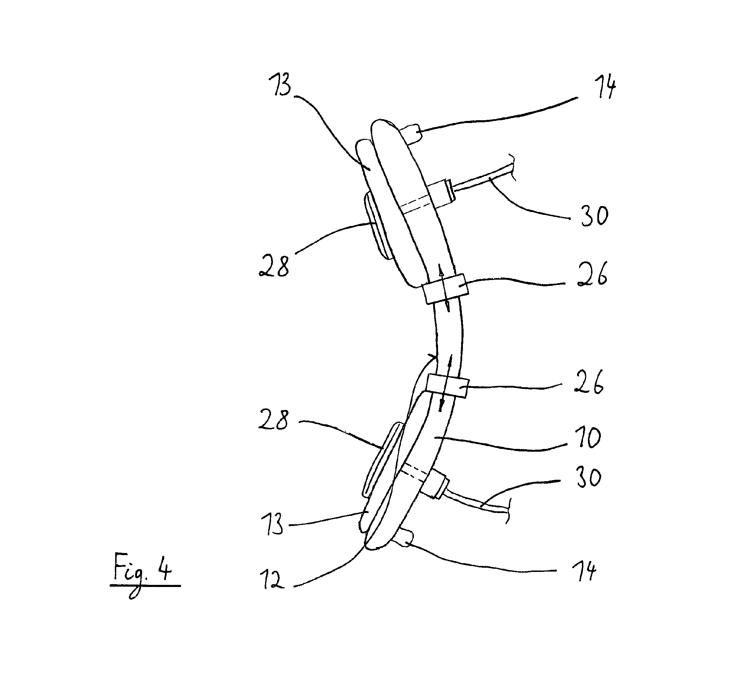

Die Druckplatte 10 hat dabei eine ovale Grundform 11 und ist an der dem Bauch des Patienten 24 zugewandten Seite konkav ausgeformt, s. Bezugsziffer 12 in den Figuren 3 und 4. Damit kann ein gut verteilter Druck auf den Bauch des Patienten vor und während des Endoskopierens aufgebaut werden.

Um diesen Druck allerdings gezielt und in einer gewünschten Weise von der Druckplatte 10 auf den Bauch des Patienten aufbringen zu können, ist - wie es aus den Figuren 3 und 4 hervorgeht - vorgesehen, dass auf der dem Bauch des Patienten zugewandten Seite der Druckplatte 10 im Ausführungsbeispiel zwei in ihrem Volumen veränderbare Polsterelemente 13 angeordnet sind.

Bei den Polsterelementen 13 handelt es sich um aufblasbare Luftkammern, die mit einer Druckluftquelle 25 in Form je eines Blasebalgs verbunden sind. Ein Manometer 29 zeigt den Druck an, der im Polsterelement 13 herrscht. Nicht dargestellt sind Ventilmittel, mit denen Luft auch wieder aus den Polsterelementen 13 abgelassen werden kann. Die genannten Elemente sind beispielsweise von Druckluftmessgeräten bekannt und müssen hier nicht weiter beschrieben werden. Hierzu gehören auch Luftschläuche 30 zwischen den Polsterelementen 13 und den Luftpumpen 25.

In Fig. 3 ist zu sehen, wie zwischen den beiden Polsterelementen 13 ein Abstandselement 27 angeordnet ist, das die beiden Polsterelemente 13 auf einem gewünschten Abstand hält. In Fig. 4 ist alternativ hierzu vorgesehen, dass zwei Klemmspangen 26 vorhanden sind, die die Druckplatte umgreifen und in Richtung der Doppelpfeile in Fig. 4 verschoben werden können, um eine gewünschte Beabstandung der Polsterelemente 13 einzustellen.

Dem Bauch des Patienten zugewandt sind auf den Polsterelementen 13 Druckübertragungselemente 28 vorgesehen, mit denen nach Art eines Handballens zielgerichtet ein gut verteilter Druck auf die Bauchoberfläche ausgeübt werden kann.

In den Figuren 2 bis 4 ist weiter zu sehen, dass Haltenoppen 14 vorgesehen werden können, um die Befestigungsgurte 7 am seitlichen Abrutschen zu hindern. Die Haltenoppen 14 sind dabei symmetrisch zu einer Längsachse L der Druckplatte 10 (s. Fig. 2) angeordnet.

Das Lösen der Befestigungsgurte 7 ist mit an sich bekannten Verschlüssen 15 sichergestellt, die ein Öffnen mit einem Handgriff erlauben.

Für die Anbringung der Befestigungsgurte 7 vor der Endoskopie können diese mit einer stabartigen Haltevorrichtung unter dem Patienten durchgezogen werden.

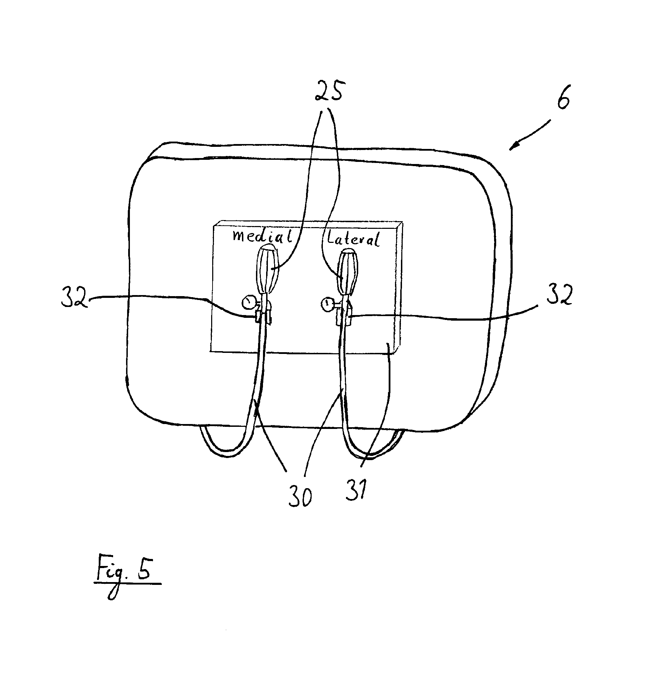

In Fig. 5 ist noch zu sehen, dass an der Rückenstütze 6, die hier nur schematisch mit ihrer Seite zu sehen ist, die vom Patienten weg gerichtet ist, eine Trägerplatte 31 angeordnet sein kann, die Halteklemmen 32 aufweist, die zur Halterung der Luftpumpen 25 bei deren Nichtbenutzung dienen. Die Luftpumpen 25 werden von den Halteklemmen bei Bedarf abgezogen und benutzt.

Demgemäß wird es möglich, einen lokalisierten Druck an den typischen Stellen des Bauchs aufbauen zu können, wozu die im Ausführungsbeispiel zwei pneumatische Druckstempel 13 dienen, die getrennt voneinander betätigt werden können, wobei die beiden Drucksysteme manuell oder elektrisch betätigt werden können; über ein Manometer 29 kann der Druck kontrolliert werden.

Somit ist in einfacher Weise eine Anpassung der optimalen Druckverhältnisse zwischen Druckplatte 10 und Bauch des Patienten möglich. Alternativ ist auch eine elektrisch bzw. elektronische Betätigung der Luftpumpe und die Steuerung bzw. Regelung des Drucks möglich.

### Bezugszeichenliste:

- 1: Vorrichtung zum Halten eines Patienten
- 2: Trägerelement
- 3: Halteelement
- 4: proximales Ende
- 5: distales Ende
- 6: Rückenstütze
- 7: Befestigungsgurt
- 8: Ende
- 9: Ende
- 10: Druckplatte
- 11: ovale Form
- 12: konkave Form
- 13: Polsterelement
- 14: Haltenoppe
- 15: Verschluss
- 16: Haltestange
- 17: Befestigungsgurt
- 18: Rolle
- 19: Endabschnitt
- 20: Endabschnitt
- 21: Tragarm
- 22: Endoskopvorschubvorrichtung
- 23: Untersuchungsliege
- 24: Patient
- 25: Druckquelle (Luftpumpe)
- 26: Klemmspange
- 27: Abstandselement
- 28: Druckübertragungselement
- 29: Manometer
- 30: Luftschlauch
- 31: Trägerplatte
- 32: Halteklemme

- L: Längsachse

## Patentansprüche

1. Vorrichtung (1) zum Halten eines Patienten während einer endoskopischen Untersuchung, umfassend:
- ein Trägerelement (2),
- ein am Trägerelement (2) angeordnetes Halteelement (3), wobei ein proximales Ende (4) des Halteelements (3) am Trägerelement (2) befestigt ist und wobei an einem distalen Ende (5) des Halteelements (3) eine Rückenstütze (6) befestigt ist,
- mindestens einen Befestigungsgurt (7) der mit seinen Enden (8, 9) an der Rückenstütze (6) lösbar befestigbar ist und den Bauch eines Patienten umfasst und
- mindestens eine Druckplatte (10), die mittels des mindestens einen Befestigungsgurts (7) umfasst und gegen die Rückenstütze (6) verspannt werden kann,
wobei auf der dem Bauch des Patienten zugewandten Seite der Druckplatte (10) mindestens ein in seinem Volumen veränderbares Polsterelement (13) angeordnet ist.

2. Vorrichtung nach Anspruch 1, **dadurch gekennzeichnet, dass** das Polsterelement (13) als aufblasbare Luftmanschette ausgebildet ist.

3. Vorrichtung nach Anspruch 1 oder 2, **dadurch gekennzeichnet, dass** das Polsterelement (13) mit einer Druckquelle (25) für ein Fluid, insbesondere für Luft, in Verbindung steht.

4. Vorrichtung nach Anspruch 3, **dadurch gekennzeichnet, dass** die Druckquelle (25) eine handbetätigte Luftpumpe oder eine elektrischer Luftpumpe ist.

5. Vorrichtung nach einem der Ansprüche 1 bis 4, **dadurch gekennzeichnet, dass** an der Druckplatte (10) mindestens eine Klemmspange (26) angeordnet ist, mit der die seitliche Erstreckung des Polsterelements (13) begrenzt werden kann.

6. Vorrichtung nach Anspruch 5, **dadurch gekennzeichnet, dass** die mindestens eine Klemmspange (26) in der Ebene der Druckplatte (10) verschieblich angeordnet ist.

7. Vorrichtung nach einem der Ansprüche 1 bis 6, **dadurch gekennzeichnet, dass** mindestens zwei Polsterelemente (13) nebeneinander auf der Druckplatte (10) angeordnet sind.

8. Vorrichtung nach Anspruch 7, **dadurch gekennzeichnet, dass** für jedes Polsterelement (13) eine Klemmspange (26) vorhanden ist, mit der die seitliche Erstreckung des Polsterelements (13) begrenzt werden kann.

9. Vorrichtung nach Anspruch 7, **dadurch gekennzeichnet, dass** zwischen zwei Polsterelementen (13) ein Abstandselement (27) angeordnet ist, das die beiden Polsterelemente (13) voneinander beabstandet hält.

10. Vorrichtung nach einem der Ansprüche 1 bis 9, **dadurch gekennzeichnet, dass** auf der dem Bauch des Patienten zugewandten Seite des Polsterelements (13) ein Druckübertragungselement (28) angeordnet ist.

11. Vorrichtung nach Anspruch 10, **dadurch gekennzeichnet, dass** das Druckübertragungselement (28) eine runde oder elliptische Form aufweist, gesehen senkrecht auf die Druckplatte (10).

12. Vorrichtung nach Anspruch 10 oder 11, **dadurch gekennzeichnet, dass** das Druckübertragungselement (28) zumindest in seiner Kontaktfläche zum Bauch des Patienten aus elastischem oder gelartigem Material besteht.

13. Vorrichtung nach einem der Ansprüche 10 bis 12, **dadurch gekennzeichnet, dass** das Druckübertragungselement (28) auf dem Polsterelement (13) aufgeklebt ist.

14. Vorrichtung nach einem der Ansprüche 3 bis 13, **dadurch gekennzeichnet, dass** an der Rückenstütze (6) eine Trägerplatte (31) angeordnet ist, die zur Halterung der mindestens einen Druckquelle (25), insbesondere der mindestens einen Luftpumpe, ausgebildet ist.

15. Vorrichtung nach Anspruch 14, **dadurch gekennzeichnet, dass** an der Trägerplatte (31) mindestens eine Halteklemme (32) für die Druckquelle (25), insbesondere für die Luftpumpe, angeordnet ist.
